(19) Europäisches Patentamt
European Patent Office
Office européen des brevets

(11) Publication number: 0 136 593
A2

(12) # EUROPEAN PATENT APPLICATION

(21) Application number: 84110644.6

(22) Date of filing: 06.09.84

(51) Int. Cl.[4]: C 07 D 213/61
C 07 D 213/64

(30) Priority: 06.09.83 US 529200
28.11.83 US 555794

(43) Date of publication of application:
10.04.85 Bulletin 85/15

(84) Designated Contracting States:
BE DE FR GB IT NL

(71) Applicant: THE DOW CHEMICAL COMPANY
Dow Center 2030 Abbott Road Post Office Box 1967
Midland Michigan 48640(US)

(72) Inventor: Burson, Richard L.
1801 Rapanos Drive
Midland Michigan 48640(US)

(74) Representative: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel Möhlstrasse 22
D-8000 München 80(DE)

(54) **Novel halopyridines and methods of making.**

(57) Novel halopyridine, e.g. 2,3-dichloro -5-iodopyridine and methyl 2-(4-((3-chloro-5-iodo-2-pyridinyl)oxy)phenoxy)-propionate, the latter being useful as an herbicide.

EP 0 136 593 A2

NOVEL HALOPYRIDINES AND METHODS OF MAKING

This invention relates to novel compounds having the formula

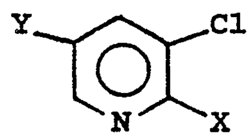

wherein y is I or Li and X is Cl, OH,

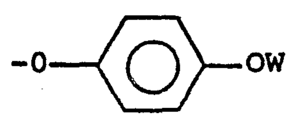

where W is H, Na, K, Mg, Ca, $N(R")_4$ or

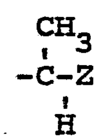

and Z represents $-CO_2H$, $-CO_2M$, $-CO_2R$, -COSR, $-CONR'_2$, $-CSNH_2$, -CN, $-CH_2OR'$ or $-CH_2O_2CR'$;

R represents $C_1$-$C_8$ alkyl or $C_3$-$C_6$ alkoxyalkyl;

each R' independently represents H or $C_1$-$C_4$ alkyl; and

each R" independently represents H, $C_1$-$C_4$ alkyl or $C_2$-$C_3$ hydroxyalkyl.

More particularly, the invention relates to 2,3-dichloro-5-iodopyridine and its use to make 2-(4-((3-chloro-5-iodo-2-pyridinyl)oxy)phenoxy) propionic acid and agriculturally acceptable derivatives thereof.

In one embodiment of the present invention, 5-bromo-2,3-dichloropyridine is lithiated by reacting it with a lithiating agent, such as, n-butyl lithium or lithium diisopropylamide, in an inert carrier medium, such as, tetrahydrofuran, dry ethyl ether or other etherial solvents, at a reduced temperature of about -70°C or lower. This lithiation reaction results in the formation, in situ, of 2,3-dichloro-5-lithiopyridine, a novel intermediate which is transitory and non-isolatable but is within the scope of the present invention. After the lithiation reaction is complete, usually in from 5 to 60 minutes, iodine ($I_2$) is added to the reaction mixture whereby 2,3-dichloro-5-iodopyridine is formed. The iodine addition is also conducted at a reduced temperature of about -70°C or lower and the iodination reaction is also usually complete in from about 5 to about 60 minutes.

The amount of inert carrier medium is not critical but it is advantageous to employ enough inert carrier medium to keep the 5-bromo-2,3-dichloropyridine in solution at reaction temperatures, generally from 10 to 20 parts by weight inert carrier medium per part by weight pyridine starting material.

The relative proportions of reactants to be employed is not critical because some of the product will be formed when employing any proportions of reactants. The reaction consumes the reactants, however, in the ratio of one mole of 5-bromo-2,3-dichloropyridine per mole of lithium and iodine. It is preferred to use an excess molar amount of lithium and iodine so that the 5-bromo-2,3-dichloropyridine starting material and 2,3-dichloro-5-lithiopyridine intermediate are completely consumed.

In carrying out this reaction, neither the rate of addition nor the order of addition of the reactants is critical. Usually, the 5-bromo-2,3-dichloropyridine and the inert carrier medium are added to an appropriate reaction vessel and cooled to a temperature less than about -70°C and preferably less than or equal to about -78°C. The lithiating agent, preferably n-butyl lithium, is then added slowly to the reaction mixture. The $I_2$ is then added, preferably immediately after formation of 2,3-dichloro-5-lithiopyridine because of the relatively unstable nature of the lithiopyridine intermediate, to the reaction mixture resulting in the formation of 2,3-dichloro-5-iodopyridine.

This reaction is typically conducted in the presence of mild agitation sufficient to maintain an essentially uniform dispersion of the reactants in the carrier medium. Ambient pressures are advantageously employed although not critical.

After completion of the reaction, the desired product is recovered and isolated employing standard separatory and purification techniques, such as solvent extraction and recrystallization.

Alternatively, this compound is prepared by reacting 5-chloro-6-hydroxynicotinic acid with $I_2$ in an aqueous alkaline iodide solution to form 3-chloro-5-iodo-2-pyridinol. This reaction is conveniently conducted at an elevated temperature of between 40°C and 150°C and preferably at about 100°C. Lowering the pH of the reaction mixture to $\leq 1$ such as, for example, by addition to the reaction mixture of $SO_2$, will precipitate the 3-chloro-5-iodo-2-pyridinol from the reaction mixture.

The 3-chloro-5-iodo-2-pyridinol is then reacted with a suitable chlorinating agent, such as, $Cl_2$, $COCl_2$ (phosgene), $PCl_5$, $POCl_3$, thionyl chloride ($SOCl_2$) or mixtures thereof, at an elevated temperature, preferably at reflux. The reaction mixture is then poured into cold water or crushed ice and the resulting precipitate isolated by filtration. The product, i.e., 2,3-dichloro-5-iodo-pyridine, may then be further purified employing standard purification and isolation techniques, such as, for example, solvent extraction and recrystallization.

In a further embodiment of the invention, 5-bromo-2,3-dichloropyridine is dissolved in ethyl ether and cooled to about -78°C. A slight excess molar quantity of n-butyl lithium, dissolved in hexane, is slowly added to the reaction mixture with mild agitation. After about 0.5 hour of stirring, a slight excess molar quantity of $I_2$, dissolved in ethyl ether, is added to reaction mixture with mild agitation. After about 0.5 hour of stirring, the reaction mixture is allowed to warm to room temperature and the 2,3-dichloro-5--iodopyridine is isolated employing standard separatory and purification techniques.

In a preferred embodiment of the invention, 3-chloro-5-iodo-2-pyridinol is reacted with an effective amount of a $PCl_5/POCl_3$ mixture at reflux until 2,3-dichloro-5-iodopyridine is formed, usually from about 1/2 to about 24 hours. The reaction mixture is then poured into crushed ice resulting in the precipitation of the desired product, i.e., 2,3-dichloro-5-iodopyridine, which is isolated and purified.

In a still further embodiment of the invention, 2-(4-[(3-chloro-5-iodo-2-pyridinyl)oxy]-phenoxy)propionic acid, and agriculturally acceptable derivatives thereof, hereinafter referred to as "the present compounds", are prepared by reacting 2,3-dichloro-5-iodopyridine with 2-(4-hydroxyphenoxy)-propionic acid, or a derivative thereof, in an inert carrier medium in the presence of a base. The resulting product is then recovered employing standard separatory and purification techniques.

A second method of preparing the present compounds is by reacting 2,3-dichloro-5-iodopyridine with hydroquinone whereby 4-((3-chloro-5-iodo-2-pyridinyl) oxy)phenol is formed. This pyridyloxyphenol is then reacted with a 2-halopropionate to form the present compounds. The 4-((3-chloro-5-iodo-2-pyridinyl)oxy)phenol intermediate is also a novel compound.

The term "agriculturally acceptable derivative", when used to describe the present compounds is meant to encompass any salt, amide, ether or ester of 2-(4--(3-chloro-5-iodo-2-pyridinyl)oxy]phenoxy propionic acid which does not substantially affect its herbicidal activity. Agriculturally acceptable derivatives include compounds of the following Formula (I)

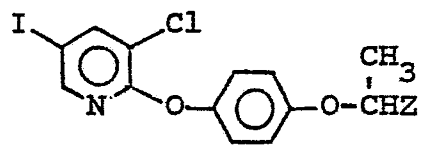

(I)

wherein Z, is as above defined.

Preferred compounds include the compounds of Formula I wherein Z represents $-CO_2H$ (the acid), $-CO_2M$ (the salts) and $-CO_2R$ (the esters). Especially preferred compounds are the esters wherein R represents methyl, ethyl, n-butyl, propyl or ethoxyethyl.

The terms "$C_1$-$C_4$ alkyl" and "$C_1$-$C_8$ alkyl" refer to different size alkyl groups which may be straight, branched, or cyclic, when the group contains at least three carbon atoms, and contain 1-4 or 1-8 carbon atoms respectively.

The terms "$C_2$-$C_3$ hydroxyalkyl" and "$C_3$-$C_6$ hydroxyalkyl" refer to different size hydroxyalkyl groups having 2-3 or 3-6 carbon atoms, respectively, and the alkyl portion may be straight or branched, or cyclic when the group contains at least three carbon atoms.

In practicing this embodiment of the invention, 2,3-dichloro-5-iodopyridine is reacted with 2-(4-hydroxyphenoxy)propionic acid, or an acceptable derivative thereof, in an inert carrier medium in the presence of a base. Suitable as an inert carrier medium are polar aprotic solvents, such as, for example, dimethylsulfoxide (DMSO), dimethylformamide (DMF), N-methylpyrrolidinone, hexamethylphosphoramide, tetrahydrofuran (THF) or sulfolane. Suitable bases include $K_2CO_3$, NaOH, KOH, sodium methoxide and $NH_4OH$. The reaction is advantageously carried out at a temperature under about 100°C and preferably about 80°C at ambient pressure. The reaction is essentially complete in from 24 to 72 hours and is advantageously conducted under an inert atmosphere such as nitrogen.

The amount of inert carrier medium is not critical but it is advantageous to employ enough inert carrier medium to keep the organic reactants in solution at reaction temperatures, generally from 1.5 to 20 parts by weight inert carrier medium per part by weight reactants. The relative proportions of reactants to be employed is not critical because some of the product will be formed when employing any proportions of reactants. The reaction consumes the reactants, however, in the ratio of one mole of 2,3-dichloro-5-iodopyridine per mole of hydroxyphenoxy propionate (or derivative) with an equivalent molar amount of base being employed to transform the hydroxyphenoxy propionate into the reactive anion form. It is preferred to employ a slight molar excess of hydroxyphenoxy propionate and

base so that the 2,3-dichloro-5-iodopyridine reactant is completely consumed.

In carrying out this reaction, neither the rate of addition nor the order of addition of the reactants is critical. Usually the inert carrier medium and base are mixed and the hydroxy-phenoxy propionate reactant is added thereafter to form the anion of the hydroxyphenoxy propionate. The 2,3-dichloro-5-iodopyridine is then added to the reaction mixture resulting in the formation of the desired product.

This reaction is typically conducted in the presence of mild agitation sufficient to maintain an essentially uniform dispersion of the reactants in the carrier medium. Ambient pressures are advantageously employed although not critical. Substantially anhydrous conditions, are preferably employed.

After completion of the reaction, the desired product is recovered and isolated employing standard separatory and purification techniques, such as solvent extraction and recrystallization.

A second method of making the present compounds is carried out by reacting 2,3-dichloro-5-iodopyridine with hydroquinone to form 4-(3-chloro-5-iodo-2-pyridinyloxy)phenol, a novel compound which is encompassed by the present invention.

A base is employed to transform hydroquinone into the reactive dianion form. Polar aprotic solvents, described hereinbefore, are employed as the inert carrier media.

4-(3-Chloro-5-iodo-2-pyridinyloxy)phenol is then reacted with a chloro- or bromo-propionate, or an acceptable derivative thereof, to form the desired compound.

The following examples further illustrate the present invention.

Example 1:

Preparation of 2,3-dichloro-5-iodopyridine

STEP A

A one liter (1 l) 3-necked round bottom flask, fitted with a mechanical stirrer, a gas sparge tube, and a dry ice/acetone condenser with a drying tube, was charged with 75.0 grams (g) (0.539 mole) of 6-hydroxynicotinic acid (obtained from Aldrich Chemical Company) and 500 milliliters (ml) of deionized water. Chlorine (45.88 g; 0.647 mole) was bubbled into the reaction over a 1.5 hour period while the temperature was kept <25°C. The resulting tan slurry was stirred for 2 hours at room temperature. A tan solid was isolated by vacuum filtration, washed with 50-100 ml of deionized water and air dried. The tan solid was further dried by placing the solid in a one liter 3-necked round bottom flask, equipped with a mechanical stirrer, a thermometer with a temperature controller, a Dean-Stark trap with a condenser and a heating mantle, and azeotropically distilling the water with toluene (∿ 700 ml charged initially). The resulting mixture was cooled to room temperature and the tan solid was isolated by filtration.

After drying overnight under aspirator vacuum at 50°C, 77.0 g (82-83% of theoretical) of solid was obtained.

STEP B

A 250 ml 3-necked round bottom flask with a thermowell was equipped with a mechanical stirrer, a dropping funnel and a condenser topped with a $N_2$ inlet and charged with 34.0 g (0.196 mole) of 5-chloro-6-hydroxynicotinic acid, from Step A, and 40 ml (67.0 g; 0.437 mole of phosphorus oxychloride) ($POCl_3$). The resulting tan slurry was heated to 105°C where the liquid refluxed briefly. Within 15 minutes, the tan solid dissolved resulting in a black solution and the refluxing ceased. The reaction mixture was stirred and heated under $N_2$ at 105°C for 2.5 hours. After the solution had cooled to room temperature, the black liquid was poured into a 2 liter 3-necked round bottom flask, fitted with a mechanical stirrer and a condenser, and charged with ice. The original reaction vessel was rinsed with water and the contents added to the second vessel. The reaction mixture was stirred overnight at ambient temperature. A solid was isolated by vacuum filtration, washed with water and air dried. The solid was transferred to a 2 liter 3-necked round bottom flask fitted with a mechanical stirrer, a thermometer and a Dean-Stark trap topped with a condenser with a $N_2$ inlet. A small amount of acetone was used to rinse the funnel and was added to the flask together with about one (1) liter of toluene. The acetone and residual water were removed by distillation. The mixture was filtered while hot to remove a small amount of black solid and gave a clear brown liquid. The brown liquid

(toluene solution) was cooled to give 23.1 g of crude product (tan solid) having a melting point of 150°-156°C. Evaporation of the toluene produced an additional 12.12 g of tan solid.

STEP C

An oven dried, 250 ml 3-necked round bottom flask, equipped with a reflux condenser topped with an $N_2$ inlet, an addition funnel, a mechanical stirrer and a thermometer with a temperature controller, was flushed with $N_2$ and charged with 12.0 g (0.0625 mole) of 5,6--dichloronicotinic acid (from Step B), 17.45 g of (0.0806 mole) of red mercuric oxide and 100 ml of $CCl_4$. The reaction mixture (a red-orange slurry) was stirred under $N_2$ at reflux, using two 175 watt infrared heat lamps as the heat source, for 2 hours. A solution of 12 g (0.075 mole) of $Br_2$ in 25 ml of $CCl_4$ was added to the refluxing reaction mixture over a 5 hour period. The reaction mixture was stirred under $N_2$ at reflux overnight. In the morning, with most of the deep red color dissipated, 40 ml of saturated $NaHCO_3$ solution was added to the cooled reaction mixture. The reaction mixture was stirred for one hour at ambient temperature and filtered through a Celite pad. The resulting precipitate was thoroughly washed with methylene chloride. The combined organic layers were washed with water (2 x 100 ml) and 100 ml of saturated NaCl solution, dried over anhydrous $MgSO_4$, filtered and evaporated to dryness. The residual light brown oil was taken up in pentane, filtered and evaporated to dryness resulting in 10.35 g of a tan oil that was 89.5% pure as indicated by the area percent on a capillary gas chromatograph (gc) (0.5 μl of neat oil) employing

standard gas chromatography (gc) procedures. The tan oil was transferred to a 50 ml miniware flask and was distilled through a short path distillation head. Only one fraction was observed that had a boiling point of 55°-65°C @ ~ 1 torr. The oil, which was ~95 percent pure as indicated by gc, crystallized to a white solid which had a melting point of 30.0°-31.0°C.

STEP D

All glassware used in Step D was dried in an oven at 100°C overnight and cooled to room temperature under $N_2$ prior to use. A 250 ml 3-necked round bottom flask, equipped with a magnetic stirrer, a thermometer, a septum, and an addition funnel topped with an $N_2$ inlet, was charged with 5.7 g (25 mmol) of 5-bromo-2,3-dichloropyridine (from Step C) and 125 ml of ethyl ether freshly distilled from sodium-benzophenone ketyl. The reaction mixture was cooled to -78°C and 16.1 ml of ~ 1.6 M n-butyl lithium in hexane was slowly added to the reaction mixture from a dried syringe. Little or no exotherm was noted although the reaction mixture immediately turned brown. The reaction mixture was stirred for 0.5 hour at -78°C and then 7.61 g (30 mmol) of $I_2$ in ~ 30 ml of freshly distilled ether was slowly added so that the temperature was maintained less than -70°C. The reaction mixture was stirred for 0.5 hour at -78°C and then allowed to warm to room temperature. The reaction mixture was poured into water and the layers were separated. The organic layer was washed with 50 ml of 1.0 N sodium thiosulfate, 50 ml of water and 50 ml of saturated NaCl solution. The organic layer was dried over anhydrous $MgSO_4$, filtered and evaporated to dryness to give a brown oil. The oil was taken up in pentane, filtered

through glass wool and evaporated to dryness resulting in 5.7 g of a brown oil that upon standing partially crystallized to needles. The mixture of oil and needles was warmed to give a homogeneous solution. The neat oil was analyzed by capillary gc and showed one major product (~ 80% by area) with a longer retention than the starting material. The oil was taken up in methanol and recrystallized to give 2.05 g of a white solid having a melting point of 56.5°C--57.5°C. The structure of the product being 2,3-dichloro-5-iodopyridine was confirmed by nuclear magnetic resonance spectra (NMR) and mass spectroscopy.

Example 2:

Preparation of 2,3-Dichloro-5-iodopyridine

STEP 1

A 250 ml 3-necked round bottom flask, fitted with a reflux condenser, thermometer, addition funnel and magnetic stirrer, was charged with 3.47 g (0.02 mole) of 5-chloro-6-hydroxynicotinic acid (from Example 1, STEP A) and 8.6 g (0.03 mole) of sodium carbonate in 90 ml of water. The reaction mixture was stirred and heated to 100°C under $N_2$. A solution of 5.1 g (0.02 mole) of iodine and 5.1 g (0.03 mole) of KI in 35 ml of water was added to the reaction mixture over 0.5 hour to minimize foaming. The reaction mixture was stirred for 1 hour at 100°C. The mixture was cooled to room temperature and the addition funnel was replaced with a gas sparge tube. $SO_2$ was passed through the mixture causing the formation of a tan precipitate. The $SO_2$ addition was maintained until the pH was <1. The slurry was stirred for 1 hour. The precipitate was isolated by

filtration, washed with water and air dried resulting in 4.05 g of a tan solid having a melting point of 202°-209°C.

STEP 2

A 50 ml 3-necked round bottom flask, equipped with a thermometer, a reflux condenser topped with a $N_2$ inlet, a stopper and a magnetic stirrer, was charged with 3.50 g (0.0137 mole) of 3-chloro-5-iodo--2-pyridinol, from STEP 1 above, 3.00 g (0.0144 mole) of phosphorus pentachloride and 5-10 ml of phosphorus oxychloride. The reaction mixture was heated to reflux and the solid dissolved resulting in a golden brown solution. The reaction mixture was stirred under $N_2$ at reflux for 4 hours and then cooled to room temperature. The reaction mixture was poured into crushed ice and allowed to stand for about 1 hour. A tan precipitate, which was shown to be product and unreacted starting material, formed and was isolated by filtration and washed with pentane. The aqueous layer was also wahsed with pentane. The combined organic layers were washed with deionized water and saturated NaCl, dried over anhydrous $MgSO_4$, filtered and evaporated to dryness to give 0.4 g of a dark oil that crystallized upon standing. The product had a melting point of 56.5-57.5°C.

Example 3:

Preparation of 2,3-Dichloro-5-iodopyridine

STEP 1

Substantially the same procedures described in STEP 1 of Example 2 were employed using 12.6 g (72.6 mmoles) of 5-chloro-6-hydroxynicotinic acid, 12.83 g (121 mmoles) of $Na_2CO_3$, 20.47 g (80.7 mmoles) of $I_2$, 20.09 g (121 mmoles) of KI and 500 ml of $H_2O$. The reaction produced 17.2 g of a tan solid.

STEP 2

A 100 ml 3-necked round bottom flask, fitted with a condenser topped with a $N_2$ inlet, a mechanical stirrer and a thermometer, was charged with 17.2 g (0.0673 mole) of 3-chloro-2-hydroxy-5-iodopyridine, 14.85 g (0.0713 mole) of $PCl_5$, and 1 ml (1.68 g; 0.0109 mole) of $POCl_3$. The reaction mixture, containing solids, was slowly stirred and slowly heated to 130°C. The reaction mixture was stirred under $N_2$ for 5 hours after which it was cooled to room temperature and allowed to stand overnight. The reaction mixture was cautiously poured into crushed ice and allowed to stand for 1 hour. Methylene chloride (200 ml) was added to the reaction mixture to dissolve the solids present in it. The layers were separated and the organic phase was washed with dilute base, water and saturated NaCl. After drying over anhydrous $MgSO_4$ and filtration, the solvent was evaporated leaving 11.8 g of a yellow solid. The solid was recrystallized from methanol resulting in 6.60 g of solid that had spectral and physical properties identical to the product obtained in Example 1, STEP D.

The compound 3-chloro-5-iodo-2-pyridinol exists in equilibrium with its tautomer, 3-chloro-5--iodo-2-pyridone. Tautomerism is a phenomenon well known to those skilled in the art and the present invention contemplates both of the above tautomers.

Example 4: Preparation of Methyl 2-(4[(3-chloro-5--iodo-2-pyridinyl)oxy]phenoxy)propionate

A 50 milliliter (ml) 3-necked, round bottom flask, fitted with a mechanical stirrer, a condenser topped with a $N_2$ inlet, and a thermometer, was charged with 6.00 grams (g) of 2,3-dichloro-5-iodopyridine (21.91 m moles), 4.73 g of methyl 2-(4-hydroxyphenoxy)-propionate (24.1 m moles), 6.05 g of anhydrous potassium carbonate (43.4 m moles) and 25 ml of dimethyl sulfoxide (DMSO). The DMSO was previously stored over molecular sieves. The reaction mixture was heated, with stirring, to 80°C under $N_2$ for 69 hours. Samples were withdrawn from the reaction mixture at 24, 48 and 69 hours. Samples were diluted with methylene chloride ($CH_2Cl_2$) and analyzed employing standard gas chromatography (gc) procedures. Percent product formation, represented as area %, was 32% (24 hour sample), 75% (48 hour sample) and 83% (69 hour sample). The heating was terminated after 69 hours and the brown mixture was allowed to cool to room temperature. Solids, were removed from the reaction mixture by filtration and washed with 100 ml of $CH_2Cl$. The filtrate was washed with 80 ml of $H_2O$, 80 ml of dilute

HCl (10%), 80 ml of 4% NaOH, 80 ml of $H_2O$ and 80 ml of saturated NaCl. The light yellow solution was dried over $MgSO_4$, filtered and rotary evaporated to dryness to give 5.39 g of a viscous oil. This viscous oil partially solidified over the weekend. Analysis by gc indicated it to be about 91% (area %) pure product. The oil was taken up in acetone and recrystallized from acetone-hexane to give 4.67 g of white solid. Analysis by gc indicated that the white solid was >99% pure.

The caustic extract from above was acidified to pH <1 with concentrated HCl to give a white solid. The white solid was taken up in about 100 ml of $CH_2Cl_2$, washed with 80 ml of $H_2O$ and 80 ml of saturated NaCl, then dried over $MgSO_4$, filtered and evaporated to dryness to give 2.00 g of tan solid which likely corresponds to 2-(4-[(3-chloro-5-iodo-2-pyridinyl)oxy]-phenoxy)propionic acid.

Example 5: Preparation of Methyl 2-(4-[(3-chloro-5--iodo-2-pyridinyl)oxy]phenoxy)propionate

Substantially the same procedures described in Example 4 were repeated using the following amounts of materials: 9.00 g of 2,3-dichloro-5-iodopyridine (32.9 m moles), 7.60 g of methyl 2-(4-hydroxyphenoxy)-propionate (38.7 m moles), 9.10 g of $K_2CO_3$ (65.8 m

moles) added to the reaction mixture initially, plus an additional 2.00 g of $K_2CO_3$ added to the reaction mixture after 42 hours, and 40 ml of DMSO. The reaction was conducted for 66 hours and resulted in the formation of 9.26 g of crude product which was shown to be 91% pure by area % gc. Recrystallization from acetone-hexane resulted in 6.75 g of product which was 98.6% pure by gc and had a melting point of 82°-84°C.

1. A compound having the formula

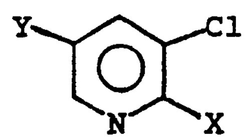

wherein Y is I or Li and X is Cl, OH,

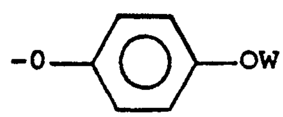

where W is H, Na, K, Mg, Ca a N $(R")_4$ or

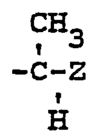

and Z represents $-CO_2H$, $-CO_2M$, $-CO_2R$, -COSR, $-CONR'_2$, $-CSNH_2$, -CN, $-CH_2OR'$ or $-CH_2O_2CR'$;

R represents $C_1$-$C_8$ alkyl or $C_3$-$C_6$ alkoxyalkyl;

each R' independently represents H or $C_1$-$C_4$ alkyl; and

each R" independently represents H, $C_1$-$C_4$ alkyl or $C_2$-$C_3$ hydroxyalkyl.

2. Compound of Claim 1 where Y is I and X is Cl.

3. Compound of Claim 1 where Y is I and X is OH.

4. Compound of Claim 1 where Y is I and X is

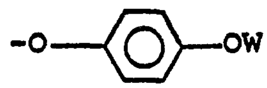

5. Compound of Claim 4 where W is H, Na or K.

6. Compound of Claim 4 where W is

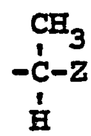

7. Compound of Claim 6 where Z is $CO_2H$.

8. Compound of Claim 6 where Z is $-CO_2CH_3$.

9. A method of preparing 2,3-dichloro-5--iodopyridine which comprises:

(a) reacting 5-bromo-2,3-dichloropyridine with an effective amount of a lithiating agent at a temperature below -70°C in an inert carrier medium to form 2,3-dichloro-5-lithiopyridine <u>in situ</u>, and then

(b) adding to the reaction mixture an effective amount of $I_2$ at a temperature below -70°C whereby 2,3-dichloro-5-iodopyridine is formed.

10. The method of Claim 2 wherein the lithiating agent is n-butyl lithium and the inert carrier medium is ethyl ether.